# EUROPÄISCHE PATENTANMELDUNG

(11) **EP 4 275 722 A1**
(43) Veröffentlichungstag der Anmeldung: **15.11.2023**
(21) Anmeldenummer: 22173096.3
(22) Anmeldetag: 12.05.2022
(51) Int. Cl.: A61M 5/32

(54) **AUTOINJEKTOR MIT PRODUKTBEHÄLTERAUFNAHMEVORRICHTUNG**

(71) Anmelder: Ypsomed AG, 3401 Burgdorf (CH)
(72) Erfinder: Kalbermatter, Gabriel, 3400 Burgdorf (CH); Bernhard, Mario, 3400 Burgdorf (CH); Schrul, Christian, 3400 Burgdorf (CH); Grünig, Nicolas, 1784 Courtepin (CH); Allenspach, Marcel, 3400 Burgdorf (CH); Tschirren, Markus, 3400 Burgdorf (CH)
(74) Vertreter: Meier Obertüfer, Jürg

(57) **Zusammenfassung**

Die Erfindung betrifft eine Produktbehälteraufnahmevorrichtung (90) sowie einen Autoinjektor umfassend - einen Spritzenhalter (12) ausgebildet zur Aufnahme einer vorgefüllten Fertigspritze (11) definierend eine Längsachse (L) welche sich von distal nach proximal erstreckt mit einem zylinderförmigem Produktbehälter (11c) welcher sich an seinem distalen Ende über eine Schulter (lld) verjüngt sowie mit einer Nadelschutzkappe (11a) und der Spritzenhalter (12) aufweisend eine distal koaxial angeordnete Führungshülse (12b), an deren Innenwand eine Sperrfläche (12a) ausgebildet ist, - einen Insert (30) aufweisend einen radial bezüglich der Längsachse aus einer relaxierten Lage auslenkbaren Schnapparm mit Schnapphaken (30a), - eine Gerätekappe (16) in welcher der Insert (30) verbindbar aufgenommen ist, wobei der Insert (30) von einer ersten in eine zweite Rastposition relativ zur Gerätekappe (16) bewegbar ist und die Verbindung der Gerätekappe (16) mit dem Insert (30) in zumindest der zweiten Rastposition rastend und formschlüssig auf Zugkräfte ausgebildet ist.

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf einen Autoinjektor mit einem Energiespeicher zur Ausschüttung einer vorgegebenen Dosis aus einem einmalig genutzten Produktbehälter.

### HINTERGRUND DER ERFINDUNG

Injektionsgeräte oder Injektionsvorrichtungen zum vereinfachten Verabreichen einer Substanz umfassen unter anderem so genannte Autoinjektoren, welche einen Energiespeicher aufweisen, mit welchem die Ausschüttung automatisch, das heisst ohne extern von einem Anwender zuzuführende oder aufzuwendende Kraft, durchgeführt werden kann. Der Energiespeicher speichert die für eine automatische Substanzabgabe erforderliche Energie vorteilhaft in mechanischer Form. Ein solcher Energiespeicher kann eine Feder sein, welche in einem gespannten Zustand in das Injektionsgerät eingebaut wird und durch Entspannen Energie abgibt. Die Energieabgabe erfolgt an eine Kolbenstange oder ein Druckelement, welches einen Kolben in einen Produktbehälter einschiebt. Der Energiespeicher kann auch vorgesehen sein um den Vorgang des Einstechens einer Injektionsnadel zu automatisieren. Alternativ kann der Einstechvorgang manuell erfolgen, also ausschliesslich durch einen Anwender, ohne hierfür in dem Injektionsgerät gespeicherte Energie zu verwenden.

Das Injektionsgerät kann einen Produktbehälterhalter zur Aufnahme eines Produktbehälters umfassen, wobei in dem Produktbehälterhalter der Produktbehälter radial, axial und vorzugsweise auch drehfest gehalten werden kann. Der Produktbehälterhalter kann mit dem Gehäuse der Injektionsvorrichtung axial- und drehfest verbunden sein, oder bei einem Einstech- und/oder Nadelrückzugsvorgang relativ zum Gehäuse bewegbar sein. Der Produktbehälter kann eine Karpule zur wiederholt lösbaren Verbindung mit Einweg-Injektionsnadeln oder eine Einweg-Fertigspritze mit einer damit unlösbar verbundenen Injektionsnadel sein. Der Produktbehälter weist einen hohlzylindrischen Produktbehälterabschnitt auf, der einen Kolben oder Stopfen verschiebbar lagert. Der Kolben kann mit dem Innenumfang des Produktbehälterabschnitts einen Dichtspalt bilden und mittels Kolbenstange in eine distale Richtung verschoben werden, um über die Injektionsnadel Produkt aus dem Produktbehälter abzugeben.

Das Injektionsgerät kann eine Nadelschutzhülse aufweisen, die nach erfolgter Injektion distal über das distale Ende der Injektionsnadel steht oder relativ zu dem Gehäuse unter Entspannung einer Nadelschutzhülsenfeder in diese Position verschoben wird, um den versehentlichen Zugriff auf die Injektionsnadel zu verhindern und ein Verletzungsrisiko zu verringern. Bei einem Autoinjektor kann die Nadelschutzhülse auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors zumindest als Sichtschutz dient.

Die Patentanmeldung WO 2016/205963 beschreibt einen beispielhaften Autoinjektor, umfassend ein Gehäuse mit Längsachse und einen axial fest im Gehäuse angeordneten Produktbehälter. Der Autoinjektor umfasst weiter eine in einer Längsrichtung zwischen einer proximalen und einer distalen Position verschiebbare Nadelschutzhülse, welche mit einer Nadelschutzfeder gekoppelt ist. Eine Spiral- oder Triebfeder, in welcher Energie für das automatische Ausschütten vom Produkt gespeichert werden kann, ist über ein erstes Ende mit dem Gehäuse und über ein zweites Ende rotationsfest mit einem koaxial zur Längsachse angeordneten Antriebselement in Form einer rotierenden Gewindestange verbunden. Die Gewindestange greift über ein Gewinde in ein im Gehäuse nicht rotierendes Vortriebsglied in Form einer Vortriebshülse, welche bei einer Verschiebung in distale Richtung den Stopfen des Produktbehälters mit einer annähernd konstanten Ausschüttgeschwindigkeit mitbewegt.

Eine Triebfeder als Antrieb zeichnet sich aus durch hohe Kräfte und ist somit geeignet für einen verzögerungsfreien Start der Ausschüttung auch bei Autoinjektoren mit einer langen Lagerdauer. Eine variable Gewindesteigung der Gewindestange kann eine variable Kennlinie der Triebfeder kompensieren um eine möglichst konstante Ausschüttkraft zu gewährleisten. Dadurch können auch grosse Ausschüttmengen von 5 ml oder mehr innerhalb von maximal 60 s gleichmässig und kontinuierlich ausgeschüttet werden. Bei einer gegenüber gängigen Autoinjektoren verlängerten Ausschüttdauer von mehr als 10 s sollte sich der Anwender jederzeit über den bestimmungsgemässen Fortgang der Injektion vergewissern können, um nicht die Injektion in unbeabsichtigter Weise vorzeitig abzubrechen.

Die Patentanmeldung WO 2012/173554 zeigt eine rotierende Anzeige am proximalen Ende eines Autoinjektors, welche durch eine Torsionsfeder während der Ausschüttung angetrieben wird. Die Anzeige umfasst mehrere Segmente mit unterschiedlichen Farben zur Signalisierung eines initialen Zustandes vor Beginn der Ausschüttung und eines finalen Zustandes nach erfolgter Ausschüttung. Die Segmente sind durch mindestens ein Fenster am proximalen Ende des Autoinjektors sichtbar, welches in seiner Ausdehnung dem Winkelbereich eines Segments entspricht.

Die Patentanmeldung veröffentlicht als CH714527A2 zeigt eine Injektionsvorrichtung mit einer Kappe zur Entfernung einer Nadelschutzkappe von einem Produktbehälter und ein Verfahren zum Montieren einer Injektionsvorrichtung, wobei die Kappe ein Eingriffselement umfasst, um beim Entfernen der Kappe von der Injektionsvorrichtung das Entfernen der Nadelschutzkappe von dem Produktbehälter zu bewirken.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter dem Begriff "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektor im Unterschied zu einem Infusionssystem die Injektionsnadel nicht über einen längeren Zeitraum von mehreren Stunden im Gewebe.

### DARSTELLUNG DER ERFINDUNG

Es ist eine Aufgabe der Erfindung, eine Anzeige für einen Autoinjektor der eingangs genannten Art zu schaffen, welche dem Anwender ein Andauern oder Fortschreiten einer Ausschüttung bei unterschiedlichsten Griffpositionen optisch anzeigen kann. Die Anzeige soll so ausgestaltet sein, dass auch bei ungünstigen, insbesondere nicht vorgesehenen Griffpositionen einer Hand des Anwenders am Autoinjektor der Anwender das Andauern der Ausschüttung erkennen kann, ohne dass er dazu die Griffposition wechseln muss. Es ist eine weitere Aufgabe einen verbesserten und dabei kostengünstigeren Autoinjektor zu schaffen. Die Montage des Autoinjektors bzw. seiner Baugruppen und das Einsetzen eines Produktbehälters anlässlich der Endmontage soll zuverlässiger möglich sein und die Bedienung bzw. Handhabung des Autoinjektors einfacher und sicherer. Die von der Endmontage des Autoinjektors zeitlich und örtlich getrennte Vormontage von Baugruppen bzw. Lagerung und Transport sowie Zuführung derselben soll einfacher und sicherer gewährleistet sein, insbesondere soll die Integrität der Baugruppen während Transport und Zuführung erhalten bleiben.

Die Aufgabe wird gelöst durch Vorrichtungen mit den Merkmalen der unabhängigen Ansprüche. Bevorzugte Ausführungsformen der Erfindung sind Gegenstand der abhängigen Ansprüche.

Ein erfindungsgemässer Autoinjektor umfasst ein einteiliges oder mehrteiliges Gehäuse mit einer Längsachse und eine vorgefüllte Fertigspritze mit einem Produktbehälter und einer daran unlösbar befestigten Injektionsnadel oder Kanüle. Die Fertigspritze ist im Gehäuse axial nicht verschiebbar aufgenommen, wobei eine Spitze der Injektionsnadel um mindestens eine Einstechtiefe in distaler Richtung über ein distales Gehäuseende vorsteht. Der Autoinjektor umfasst weiter eine zur einmaligen Ausschüttung eines maximalen Inhalts des Produktbehälters vorgespannte Torsionsfeder, ein Antriebselement, ein Vortriebselement, und eine Nadelschutzhülse. Zur Ausschüttung von Flüssigkeit aus dem Produktbehälter durch die Injektionsnadel versetzt die Torsionsfeder das Antriebselement in Rotation um die Längsachse, und das rotierende Antriebselement bewirkt eine Linearbewegung des Vortriebselements zur Verschiebung eines Kolbens im Produktbehälter. Die Nadelschutzhülse wird beim Anpressen des Autoinjektors an eine Injektionsstelle und dem dadurch bewirkten Einstechen der Injektionsnadel in die Injektionsstelle um einen Betätigungshub in proximale Richtung bewegt und startet oder ermöglicht dadurch eine Ausschüttung von Flüssigkeit. Der Betätigungshub der Nadelschutzhülse entspricht dabei mindestens der Einstechtiefe der Injektionsnadel.

Der Autoinjektor umfasst schliesslich eine Anzeige zur Signalisierung des Andauerns einer Ausschüttung, aufweisend ein durch das Antriebselement angetriebenes, um die Längsachse rotierendes Anzeigeelement mit einem optischen Kontrastmuster, und ein Fenster am proximalen Ende des Autoinjektors, durch welches das rotierende Kontrastmuster sichtbar ist. Das Fenster ist um die Längsachse vollständig um 360° umlaufend, also nicht durch einen Rahmen oder Steg parallel zur Längsachse unterbrochen, welcher einen toten Winkel für die Sicht auf das Anzeigeelement erzeugt. Das Fenster ist aus einem transparenten und bevorzugt stabilen oder tragfähigen Material gefertigt. Solange ein noch so kleiner Teil des Fensters sichtbar bleibt, also auch wenn der Anwender ungeschickterweise einen überwiegenden Teil des Fensters mit seiner den Autoinjektor haltenden Hand abdeckt, beispielsweise indem er mit dem Handballen am proximalen Ende abstützt, kann das rotierende Anzeigeelement erkannt werden.

Das Kontrastmuster ist höchstens diskret rotationssymmetrisch um die Längsachse, aber nicht kontinuierlich rotationssymmetrisch, damit eine Rotation des Kontrastmusters auch erkannt werden kann. Das Kontrastmuster umfasst mindestens eine Fläche oder ein graphisches Element in einem von einer Hintergrundfarbe verschiedenem Farb- oder Grauton, bevorzugt in Form von sich in Rotationsrichtung wiederholenden graphischen Elementen, wie beispielsweise parallelen Linien.

Ein Autoinjektor nach der Erfindung ist geeignet zur Ausschüttung der im Produktbehälter enthaltenen Produktmenge während einer gegenüber bekannten Autoinjektoren erhöhten Ausschüttdauer von mehr als 10 s, bevorzugt mehr als 20 s oder 30 s. Dadurch ist auch bei Produktmengen von über 2.25 ml und von bevorzugt mindestens 3 ml oder 4 ml eine kontinuierliche oder durchschnittliche Ausschüttrate relativ gering, so dass die injizierte Menge an Medikament laufend vom subkutanen Gewebe aufgenommen oder resorbiert werden kann. Das Einhalten einer minimalen Haltezeit zwischen Ausschüttende und Wegbewegen des Autoinjektors von der Einstichstelle ist daher weniger kritisch. Entsprechend ist keine optische, akustische, oder taktile Signalisierung vonnöten, welche dem Anwender das Ende einer Haltezeit anzeigt.

In einer bevorzugten Ausführungsform ist das rotierende Anzeigeelement direkt oder unmittelbar, insbesondere ohne Getriebe, durch das Antriebselement angetrieben. Das Anzeigeelement rotiert also mit der derselben Drehgeschwindigkeit wie das Antriebselement.

In einer weiter bevorzugten Ausführungsform führt das Anzeigeelement während der Ausschüttung weniger als eine Umdrehung, bevorzugt weniger als eine halbe Umdrehung oder gar weniger als ein Drittel einer Umdrehung pro Sekunde aus. Dadurch wird sichergestellt, dass die Drehbewegung des Kontrastmusters von Auge gut verfolgbar ist und nicht etwa für den Betrachter zu einem unbestimmten rotationssymmetrischen Muster verwischt.

In einer bevorzugten Ausführungsform ist weder am Fenster noch am anschliessenden Gehäuse eine optische Markierung zur Feststellung einer relativen Drehposition des Kontrastmusters vorgesehen. Durch das Anzeigeelement erfolgt keine Anzeige eines Anfangs- oder Endzustands, auch kein Relativangabe zum Fortschritt der Ausschüttung, es dient ausschliesslich zur Signalisierung des Fortschreitens oder Andauerns der Ausschüttung.

In einer bevorzugten Ausführungsform sind das rotierende Anzeigeelement und das Gehäuse oder das Fenster zur Erzeugung eines Ausschüttgeräusches ausgebildet, wodurch dem Anwender zusätzlich das Andauern des Ausschüttvorganges akustisch signalisiert wird. Bevorzugt ist das Ausschüttgeräusch ein kontinuierliches Klickgeräusch, welches durch ein Raster am Fenster oder am Gehäuse und ein darin eingreifendes radial oder axial flexibles Eingriffselement am Anzeigeelement, oder umgekehrt durch ein rotierendes Raster am Anzeigeelement und ein stationäres Eingriffselement erzeugt wird. Auf eine separate optische, akustische, und/oder taktile Signalisierung des Ausschüttendes, beispielsweise durch ein mechanisch oder elektronisch erzeugtes Signal, kann in diesem Fall wie auch bei einer rein optischen Bewegungsanzeige verzichtet werden.

In einer bevorzugten Ausführungsform weist das Fenster einen ersten, zylinderförmigen Bereich parallel zur Längsachse, und einen zweiten, in proximaler Richtung nahtlos an den ersten anschliessenden Bereich mit stetig oder kontinuierlich abnehmendem Durchmesser auf. Der erste Bereich ermöglicht eine Sicht auf das Anzeigeelement aus jeder seitlichen Blickrichtung senkrecht zur Längsachse, und der zweite Bereich ermöglicht eine Sicht auf das Anzeigeelement in distaler Blickrichtung von hinten, beziehungsweise bei senkrecht gehaltenem Autoinjektor von oben. Der zweite Bereich ist in Richtung proximal abgerundet oder einschnürend ausgebildet, und kann in einem dritten Bereich senkrecht zur Längsachse enden. Das Anzeigeelement kann eine dem Verlauf des Fensters folgende konvexe Anzeigefläche aufweisen oder eine kegelmantelförmige Anzeigefläche mit einer mittleren Neigung bezüglich der Längsachse. Bevorzugt ist das Fenster bezüglich der Längsachse rotationssymmetrisch, also mit kreisförmigen Querschnitten in Schnittebenen senkrecht zur Längsachse.

In einer vorteilhaften Variante umfasst der Autoinjektor eine Nadelschutzhülse, welche im Auslieferzustand des Autoinjektors distal über das Gehäuse vorsteht und durch eine Nadelschutzfeder in distale Richtung vorgespannt ist. Bei Anpressen des Autoinjektors an eine Injektionsstelle führt die Nadelschutzhülse eine Betätigungsbewegung in proximale Richtung aus und bei Entfernen des Autoinjektors von der Injektionsstelle eine Nadelschutzbewegung in distaler Richtung, um mit einem hülsenförmigen, bevorzugt rotationssymmetrischen Abschnitt die Injektionsnadel seitlich zu umgeben und radial abzudecken. Die Nadelschutzhülse weist an einem distalen Ende einen ringförmigen Flansch oder Fuss auf als vergrösserte Auflagefläche zum Gewebe um die Injektionsstelle. Der Flansch ist permanent mit dem hülsenförmigen Abschnitt verbunden und bevorzugt einstückig mit diesem ausgebildet. Ein maximaler Durchmesser des Flansches ist grösser als ein maximaler Durchmesser des hülsenförmigen Abschnitts.

Bevorzugt ist der äussere Rand oder die Peripherie des Flansches an die Form einer distalen Öffnung im Gehäuse angepasst. Im eingestochenen Zustand bildet der Flansch somit einen Abschluss des Gehäuses. Weiter bevorzugt ist der Flansch konkav gewölbt ausgebildet, so dass der äussere Rand sich weiter distal befindet als der Übergang zwischen Flansch und hülsenförmigem Abschnitt. Gegenüber einem Flansch mit einer streng ebenen, ringförmigen Auflagefläche mit identischem Aussendurchmesser ist dadurch erstens ein Verkippen des aufgesetzten Autoinjektors weniger wahrscheinlich, und zweitens führt die Konzentration der Druckbelastung auf die von der Einstichstelle am weitesten entfernte Peripherie des Flansches zu weniger Gegendruck im Gewebe und somit zu weniger Schmerz beim Anwender.

Weiter bevorzugt umfasst der Autoinjektor eine Gewindestange als Antriebselement und eine Vortriebshülse mit Innengewinde als Vortriebselement, alternativ eine Antriebshülse mit Innengewinde als Antriebselement und eine Gewindestange als Vortriebselement, wobei das Vortriebselement eine Nut oder einen Nocken als Axialführungselement für eine ausschliesslich lineare Vortriebsbewegung im Gehäuse aufweist. Der Autoinjektor ist bevorzugt dimensioniert zur Aufnahme einer vorgefüllten Fertigspritze umfassend den Produktbehälter und die Injektionsnadel, und aufweisend ein Füllvolumen von mindestens 3 ml, bevorzugt mindestens 5 ml.
Zusammengefasst und in anderen Worten kann ein Autoinjektor nach Aspekten der Erfindung wie folgt ausgeführt sein:
Autoinjektor umfassend
   - ein Gehäuse (10a, 10b) mit einer Längsachse L und zur Aufnahme eines Produktbehälters (11),
   - ein Vortriebsglied (22) und ein rotierendes Antriebselement (21) zur Bewegung des Vortriebsglieds (22) in Längsrichtung und zur automatischen Ausschüttung eines in dem Produktbehälter (11) enthaltenen flüssigen Produkts durch eine Injektionsnadel (11b),
   - eine Anzeige zur Signalisierung des Andauerns einer Ausschüttung, umfassend ein durch das Antriebselement (21) angetriebenes rotierendes Anzeigeelement (25a) mit einem optischen Kontrastmuster (), und umfassend ein Fenster (25b) am proximalen Ende des Autoinjektors, durch welches das rotierende Kontrastmuster sichtbar ist, dadurch gekennzeichnet, dass das Fenster (25b) ein um die Längsachse vollständig umlaufendes Fenster ist.
Autoinjektor wobei das rotierende Anzeigeelement (25a) direkt durch das Antriebselement (21) angetrieben ist.
Autoinjektor wobei das Anzeigeelement (25a) während einer Ausschüttung weniger als eine, bevorzugt weniger als eine halbe Umdrehung pro Sekunde ausführt.
Autoinjektor wobei keine optische Markierung zur Feststellung einer Drehposition des Kontrastmusters vorgesehen ist.
Autoinjektor wobei das rotierende Anzeigeelement (25a) zur Erzeugung eines Ausschüttgeräusches ausgebildet ist.
Autoinjektor wobei das Fenster (25b) einen ersten, zylinderförmigen Bereich parallel zur Längsachse, und einen zweiten, in proximaler Richtung nahtlos an den ersten anschliessenden Bereich mit stetig abnehmendem Durchmesser aufweist.
Autoinjektor weiter umfassend
   - eine Nadelschutzhülse (14), welche im Auslieferzustand des Autoinjektors distal über das Gehäuse vorsteht, wobei bei Anpressen des Autoinjektors an eine Injektionsstelle die Nadelschutzhülse (14) eine Betätigungsbewegung in proximale Richtung ausführt und bei Entfernen des Autoinjektors von der Injektionsstelle eine Nadelschutzbewegung in distaler Richtung ausführt, um mit einem hülsenförmigen Abschnitt (14b) die Injektionsnadel (11b) seitlich zu umgeben,
   wobei die Nadelschutzhülse (14) an einem distalen Ende einen ringförmigen Flansch (14c) zum Kontakt mit der Injektionsstelle aufweist, mit einem maximalen Durchmesser, welcher grösser ist als der maximale Durchmesser des hülsenförmigen Abschnitts (14b).
Autoinjektor wobei ein äusserer Rand des Flanschs (14c) an eine distale Öffnung im Gehäuse (10b) angepasst ist.
Autoinjektor wobei der Flansch (14c) konkav gewölbt ausgebildet ist.
Autoinjektor wobei das Antriebselement (21) eine Gewindestange ist und das Vortriebselement (22) eine Vortriebshülse mit einem Axialführungselement für eine ausschliesslich lineare Vortriebsbewegung im Gehäuse (10b) ist.
Autoinjektor weiter aufweisend eine vorgefüllte Fertigspritze umfassend den Produktbehälter (11) und die Injektionsnadel (11b), und aufweisend ein Füllvolumen von mindestens 3 ml, bevorzugt mindestens 5 ml.

Weitere bevorzugte Ausführungsformen der Erfindung, insbesondere von Produktbehälteraufnahmevorrichtungen für einen verbesserten Autoinjektor werden nachfolgend beschrieben.

In einer bevorzugten Ausführungsform umfasst eine Produktbehälteraufnahmevorrichtung für einen Autoinjektor einen Spritzenhalter ausgebildet zur Aufnahme einer vorgefüllten Fertigspritze definierend eine Längsachse welche sich von distal nach proximal erstreckt mit einem zylinderförmigem Produktbehälter welcher sich an seinem distalen Ende über eine Schulter verjüngt sowie mit einer Nadelschutzkappe wobei der Spritzenhalter eine distal koaxial angeordnete Führungshülse aufweist, an deren Innenwand eine Sperrfläche ausgebildet ist, insbesondere kann die Sperrfläche auch durch einzelne oder verbundene Rippen oder Speichen gebildet sein. Weiter einen hülsenförmigen Insert aufweisend einen radial bezüglich der Längsachse aus einer relaxierten Lage auslenkbaren Schnapparm mit Schnapphaken, sowie eine Gerätekappe in welcher der Insert verbindbar aufgenommen ist, wobei der Insert von einer ersten in eine zweite Rastposition relativ zur Gerätekappe bewegbar ist und die Verbindung der Gerätekappe mit dem Insert in zumindest der zweiten Rastposition rastend und formschlüssig auf Zugkräfte entlang der Längsachse ausgebildet ist, wobei der Schnapparm mit Schnapphaken in der ersten Rastposition radial auslenkbar ist und in der zweiten Rastposition durch die Sperrfläche an einer radialen Auslenkung gehindert wird.

Bevorzugt ist die Produktbehälteraufnahmevorrichtung weitergebildet indem der Schnapparm mit Schnapphaken in der ersten Rastposition des Inserts durch die Nadelschutzkappe radial nach Aussen auslenkbar ist. Dadurch sind die Fügekräfte bei der Montage der Fertigspritze minimal und die Verschlusssicherheit des Produktbehälters wird nicht kompromittiert.

Bevorzugt ist die Produktbehälteraufnahmevorrichtung weitergebildet indem der Schnapparm mit Schnapphaken dazu ausgebildet ist, in der zweiten Rastposition des Inserts die Nadelschutzkappe in distale Richtung formschlüssig axial mitzunehmen. Dies erlaubt eine sichere Trennung der Nadelschutzkappe beim Abziehen von der Spritze und es ergeben sich keine unnötig hohen Abzugskräfte durch zusätzliche Reibung.

Bevorzugt ist die Produktbehälteraufnahmevorrichtung weitergebildet indem der Spritzenhalter so ausgebildet ist, dass ein distaler Abschnitt der Fertigspritze insbesondere die Nadelschutzkappe in der Führungshülse axial bewegbar ist und diese Bewegbarkeit der Fertigspritze in distaler Richtung formschlüssig begrenzt ist. Dadurch wird die Fertigspritze bei der Montage radial zentriert und kann spielfrei am vorgesehenen axialen Ort in der Vorrichtung positioniert werden.

Bevorzugt ist die Produktbehälteraufnahmevorrichtung weitergebildet indem die Führungshülse mit ihrem proximalen Ende einen Anschlag bildet für den zylinderförmigen Produktbehälter insbesondere für dessen Schulter. Dadurch wird die Fertigspritze bei der Montage am vorgesehenen axialen Ort in der Vorrichtung positioniert.

Bevorzugt umfasst die Produktbehälteraufnahmevorrichtung weiter eine Nadelschutzhülse mit einem Halteschnapper wobei in der ersten Rastposition der Halteschnapper den Insert durch Eingriff in die erste Verschnappung kraftschlüssig relativ zur Nadelschutzhülse verbindet und/oder in der zweiten Rastposition der Halteschnapper den Insert durch Eingriff in die zweite Verschnappung oder Bewegung in der ersten Verschnappung kraftschlüssig relativ zur Nadelschutzhülse verbindet. Dadurch wird die Nadelschutzhülse bzw. der Insert im Autoinjektor in vorgesehener axialer Position und Drehlage gehalten und die Haltekraft erhöht auch die Sicherheit gegen unbeabsichtigtes Auslösen durch hohe Beschleunigungen, beispielsweise beim Transport oder wenn das Gerät zu Boden fällt.

Bevorzugt ist die Produktbehälteraufnahmevorrichtung weitergebildet indem die Gerätekappe von der Produktbehälteraufnahmevorrichtung durch eine Abzugsbewegung trennbar ist, wobei der Insert mitgenommen wird und die kraftschlüssige Verbindung zwischen Nadelschutzhülse und Insert gelöst wird. Dadurch ergibt sich ein definierter Anfangswiderstand, welcher unbeabsichtigtes Lösen oder ungewünschtes Spiel reduziert.

Bevorzugt umfasst die Produktbehälteraufnahmevorrichtung weiter ein Gehäuse oder Gehäuseteil wobei der Spritzenhalter unbeweglich zumindest auf axialen Zug in distaler Richtung oder axialen Druck aus proximaler Richtung in diesem aufgenommen ist oder einstückig mit diesem gebildet ist. Damit wird sichergestellt, dass die der Spritzenhalter beim Abziehen der Nadelschutzkappe oder bei der Ausschüttung sicher am vorgesehenen axialen Ort in der Vorrichtung gehalten wird.

Bevorzugt ist die Produktbehälteraufnahmevorrichtung in einer weiteren Ausführungsform der Erfindung gebildet indem der Insert von der ersten in die zweite Rastposition relativ zur Gerätekappe axial von proximal nach distal bewegbar ist und durch eine Rippe in einer Führungsnut drehfest geführt ist und/oder die Schnappnocke von der ersten Verschnappung in die die zweite Verschnappung wechselt. Dadurch ist der Insert in einer Unterbaugruppe der Vorrichtung vor der Montage der Fertigspritze unverlierbar in einer Vormontageposition gesichert und kann während der Montage der Fertigspritze in seiner Endmontageposition gesichert werden. Dabei kann die Bewegung des Insert durch ein Werkzeug oder durch ein anderes Teil, welches bei der Montage axial bewegt bewirkt werden. Insbesondere kann die Bewegung von der ersten in die zweite Rastposition aus der Fügebewegung der Fertigspritze bewirkt bzw. abgeleitet werden.

Bevorzugt ist die Produktbehälteraufnahmevorrichtung weitergebildet indem der Insert über eine proximal vorgesehene Stirnseite durch die Schulter axial in distale Richtung drängbar ist. Dadurch wird der Insert direkt durch die axiale Fügebewegung der Fertigspritze bei der Montage in seine Endmontageposition gebracht und es kann auf ein komplexes Werkzeug, welches ins Innere der Vorrichtung greifen müsste verzichtet werden.

Bevorzugt ist die Produktbehälteraufnahmevorrichtung weitergebildet indem die Sperrfläche die an der Innenwand der Führungshülse ausgebildet ist auf einen axialen Abschnitt begrenzt ist. Dieser Abschnitt ist so gewählt, dass am Anfang der Fügebewegung der Fertigspritze bei der Montage der Schnapparm mit Schnapphaken durch das Aussenprofil der Nadelschutzkappe frei sowie reibungsarm radial ausgelenkt werden kann bis er elastisch hinter dem proximalen Rand der Nadelschutzkappe einfedert und gegen Ende bzw. nach der Fügebewegung formschlüssig in dieser Lage durch die Sperrfläche oder eine andere geeignete Sperrgeometrie gegen radiales Ausbrechen gesichert bleibt.

Bevorzugt ist die Produktbehälteraufnahmevorrichtung in einer weiteren Ausführungsform der Erfindung gebildet indem der Insert von der ersten in die zweite Rastposition relativ zur Gerätekappe drehend um die Längsachse bewegbar ist und durch eine Schnappnocke in einer Schnappnut axial fest geführt ist und/oder durch die Nut in der zweiten Rastposition drehfest sicherbar ist. Dadurch ist der Insert in einer Unterbaugruppe der Vorrichtung vor der Montage der Fertigspritze unverlierbar in einer Vormontageposition gesichert und kann während der Montage der Fertigspritze in seiner Endmontageposition gesichert werden.

Bevorzugt ist die Produktbehälteraufnahmevorrichtung weitergebildet indem der Insert über einen oder mehrere distal vorgesehene Montagenocken drehend um die Längsachse bewegbar ist. Über die Montagenocken kann temporär ein Drehmoment mittels einem Drehwerkzeug oder mittels statischen Strukturen in der Montagevorrichtung (gegenüber denen die Vorrichtung gedreht werden kann) eingekoppelt werden, welches die Drehbewegung bewirkt. Dazu sind Durchbrüche in der Gerätekappe vorgesehen.

Bevorzugt ist die Produktbehälteraufnahmevorrichtung weitergebildet indem die Sperrfläche die an der Innenwand der Führungshülse ausgebildet ist sektorweise eine oder mehrere Ausnehmungen aufweist. Diese Ausnehmung ist so gewählt, dass während der Fügebewegung der Fertigspritze bei der Montage der Schnapparm mit Schnapphaken durch das Aussenprofil der Nadelschutzkappe frei radial ausgelenkt werden kann bis er elastisch hinter dem proximalen Rand der Nadelschutzkappe einfedert und nach der Fügebewegung die Drehbewegung des Insert wie oben beschrieben vorgenommen wird, so dass der Schnapparm mit Schnapphaken formschlüssig in dieser Lage durch die Sperrfläche oder eine andere geeignete Sperrgeometrie gegen radiales Ausbrechen gesichert bleibt.

Bevorzugt umfasst ein erfindungsgemäss weitergebildeter Autoinjektor für die Verabreichung eines flüssigen Produkts
- eine erfindungsgemässe Produktbehälteraufnahmevorrichtung
- ein Vortriebsglied und ein Antriebselement zur Bewegung des Vortriebsglieds in Längsrichtung und zur automatischen Ausschüttung eines in dem Produktbehälter der vorgefüllten Fertigspritze enthaltenen flüssigen Produkts durch eine Injektionsnadel wobei die Nadelschutzhülse, welche im Auslieferzustand des Autoinjektors distal über das Gehäuse vorsteht, bei Anpressen des Autoinjektors an eine Injektionsstelle eine Betätigungsbewegung in proximale Richtung ausführt und bei Entfernen des Autoinjektors von der Injektionsstelle eine Nadelschutzbewegung in distaler Richtung ausführt, um mit einem hülsenförmigen Abschnitt die Injektionsnadel seitlich zu umgeben.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
- Fig.1: die Komponenten eines Autoinjektors,
- Fig.2: zwei Längsschnitte durch den Autoinjektor aus Fig.1 vor der Injektion,
- Fig.3: zwei Ansichten des Anzeigeelements,
- Fig.4: eine Ansicht des distalen Endes der Nadelschutzhülse.
- Fig.5: die Komponenten einer ersten Ausführungsform einer Produktbehälteraufnahmevorrichtung
- Fig.6: je zwei Längs- und Querschnitte der Vorrichtung aus Fig.5 im Zustand vor der Endmontage,
- Fig.7: je ein Längs- und Querschnitt der Vorrichtung aus Fig.5 im Zustand des Fügens der Fertigspritze,
- Fig.8: zwei Längs- und ein Querschnitt der Vorrichtung aus Fig.5 im Zustand nach der Sperrbewegung des Inserts
- Fig.9: zwei Längs- und ein Querschnitt der Vorrichtung aus Fig.5 im Zustand nach dem Abziehen der Nadelschutzkappe
- Fig.10: die Komponenten einer zweiten Ausführungsform einer Produktbehälteraufnahmevorrichtung
- Fig.11: je zwei Längs- und Querschnitte der Vorrichtung aus Fig.10 im Zustand vor der Endmontage,
- Fig.12: je ein Längs- und Querschnitt der Vorrichtung aus Fig.10 im Zustand des Fügens der Fertigspritze,
- Fig.13: zwei Längs- und ein Querschnitt der Vorrichtung aus Fig.10 im Zustand nach der Sperrbewegung des Inserts; und
- Fig.14: zwei Längs- und ein Querschnitt der Vorrichtung aus Fig.10 im Zustand nach dem Abziehen der Nadelschutzkappe

### FIGURENBESCHREIBUNG

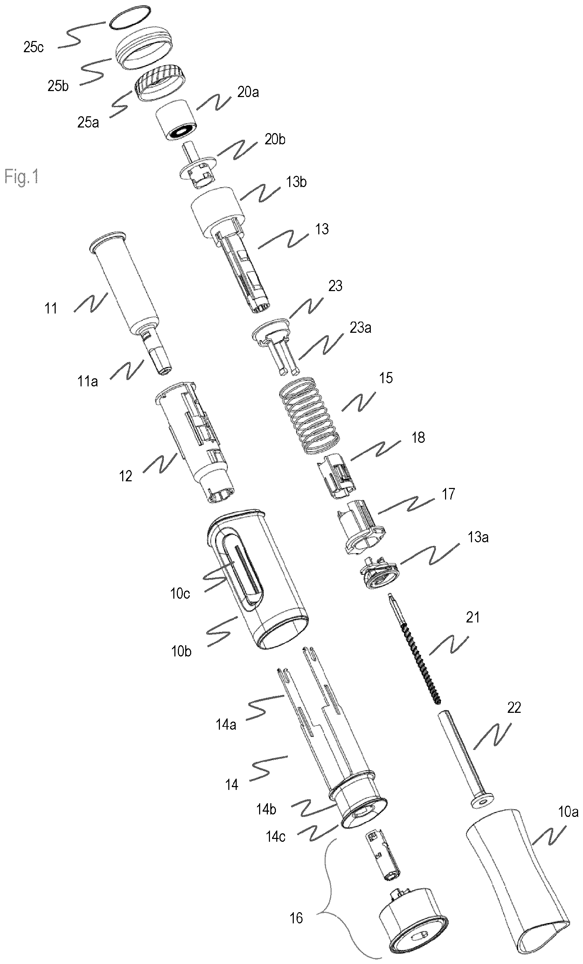

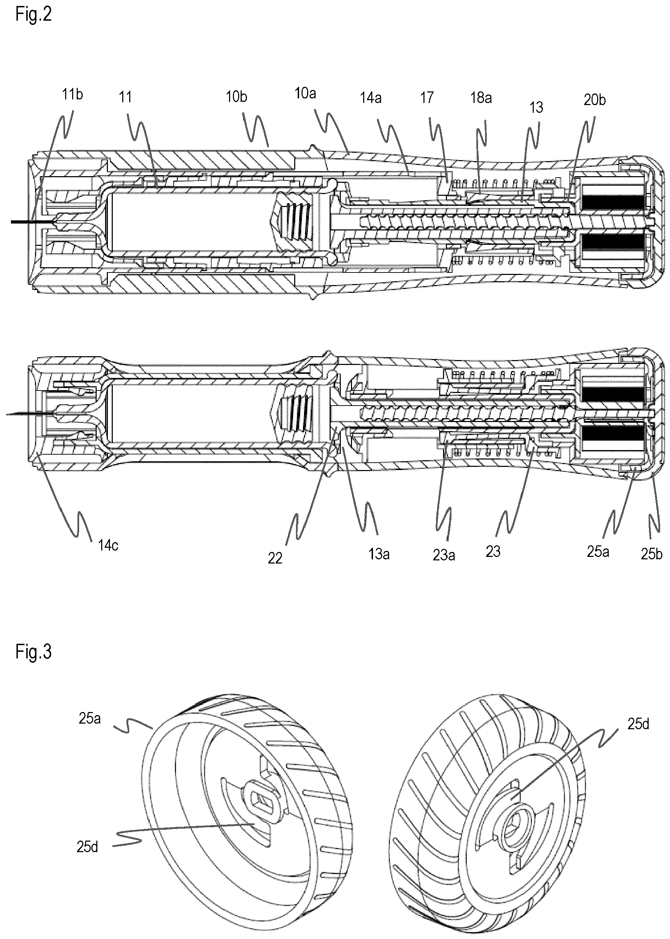

Fig.1 ist eine Explosionsdarstellung der Komponenten eines Autoinjektors nach der Erfindung, und Fig.2 zeigt zwei zueinander um die Längsachse um 90° gedrehte Längsschnitte durch den Autoinjektor nach Fig.1 im eingestochenen Zustand und bereit zur Ausschüttung.

Der Autoinjektor weist ein hülsenförmiges, längliches Gehäuse auf mit einer Längsachse L und umfassend ein distales Gehäuseteil 10b und ein damit unlösbar verschnapptes proximales Gehäuseteil 10a in Form eines Griffs. Ein Produktbehälter in Form einer Fertigspritze 11 mit einer am Produktbehälter unlösbar befestigten Injektionsnadel ist in einem Spritzenhalter 12 gehalten, wobei der Spritzenhalter im Gehäuse axial- und drehfest aufgenommen ist. Die Fertigspritze 11 wird von einem fest im Gehäuseteil 10a verankerten Haltefederabschnitt 13a in distale Richtung in einen Eingriff mit einer Schulter des Spritzenhalters 12 gedrückt. Die Fertigspritze 11 ist in Bezug auf das Gehäuseteil 10b so angeordnet, dass die Spitze der Injektionsnadel um eine der subkutanen oder intramuskulären Einstechtiefe entsprechende Länge über das distale Ende einer proximalen Zwischenposition der Nadelschutzhülse 14 hinausragt und durch eine Nadelschutzhülse 14 vor und nach der Injektion zumindest seitlich geschützt oder abgedeckt wird. Die Nadelschutzhülse 14 wird beim Einstechen der Injektionsnadel in die Injektionsstelle entlang der Längsachse L um einen Betätigungshub und gegen die Kraft einer Nadelschutzfeder 15 in proximale Richtung geschoben und löst dadurch eine Produktausschüttung aus. Die Nadelschutzhülse umfasst dazu zwei Hülsenarme 14a, welche gegenüber zwei als Sichtfenster bezeichneten Ausnehmungen 10c des Gehäuses um 90° um die Längsachse L versetzt beziehungsweise verdreht angeordnet sind. Nach der erfolgten Injektion kann die Nadelschutzhülse 14 relativ zu dem Gehäuseteil 10b aus der betätigten Position entlang der Längsachse L in die distale Richtung in eine Nadelschutzposition verschoben und dort gegen erneutes Zurückschieben blockiert werden. Die Nadelschutzhülse umfasst einen hülsenförmigen oder hohlzylindrischen Abschnitt 14b und einen Flansch 14c am distalen Ende. Der Querschnitt des hohlzylindrischen Abschnitts 14b ist oval, genauso wie der äussere Umfang des Flansches, so dass vorliegend der Flansch eine konstante Breite oder radiale Ausdehnung aufweist.

Ein Federpaket umfasst eine Spiralfeder 20a und eine Federspule 20b. Die Spiralfeder 20a ist mit ihrem äusseren Ende drehfest an einer Federhülse 13b als Teil eines fest im Gehäuse verankerten Mechanikhalters 13 befestigt. Das innere Ende der Spiralfeder 20a ist drehfest mit der Federspule 20b verbunden. Die Federspule 20b umfasst einen Federschaft und einen distalen Federflansch. Die Spiralfeder 20a beziehungsweise die Federspule 20b versetzt ein Antriebselement 21 in eine Rotationsbewegung und ein Vortriebselement 22 in eine bevorzugt rein axiale Vortriebsbewegung. Dazu greift ein Gewindeelement in ein sich über den Ausschütthub erstreckendes Gewinde mit einer variablen Gewindesteigung.

Die Fertigspritze 11 umfasst einen zylindrischen Spritzenkörper als Produktbehälter, an dessen distalen Ende eine hohle Injektionsnadel mit einer Spritzenschulter fest verbunden ist. Die Injektionsnadel der Fertigspritze ist von einer Nadelschutzkappe 11a abgedeckt, welche als sogenanntes Rigid Needle Shield (RNS) ausgebildet ist und ein gummielastisches Nadelschutzelement und eine Hülle aus Hartkunststoff umfasst. Die Nadelschutzkappe schützt die Injektionsnadel gegen mechanische Einwirkungen und Verschmutzung, und hält die Injektionsnadel und das Produkt steril. An dem distalen Ende des Autoinjektors ist in seinem Auslieferungszustand eine zweiteilige Geräte- oder Abziehkappe 16 angeordnet, die vor der Verwendung des Autoinjektors zusammen mit der Nadelschutzkappe 11a axial abgezogen und/oder abgedreht und vollständig entfernt wird.

Eine Schalthülse 17 ist formschlüssig mit einem proximalen Ende der Hülsenarme 14a der Nadelschutzhülse 14 und mit einem distalen Ende der Nadelschutzfeder 15 angeordnet und zumindest teilweise von Letzterer umgeben. Die Schalthülse 17 ist bevorzugt mit dem proximalen Ende der Hülsenarme der Nadelschutzhülse 14 verschnappt. Die Bewegung der Schalthülse 17 in distale Richtung ist durch den Haltefederabschnitt 13a begrenzt, welcher seinerseits nach der Montage der Schalthülse 17 mit dem Mechanikhalter 13 verschnappt. Innerhalb und koaxial zur Schalthülse 17 ist eine Sperrhülse 18 angeordnet, welche über ein sägezahnförmiges, an einem in distale Richtung weisenden Arm federnd angebrachtes Verriegelungsglied 18a derart an die Schalthülse 17 gekoppelt ist, dass eine Betätigungsbewegung von Nadelschutzhülse 14 und Schalthülse 17 auch die Sperrhülse 18 nach proximal bewegt. Durch einen zusätzlichen proximalen Arretierhub der Sperrhülse 18 relativ zur Schalthülse 17 in eine proximale Endposition wird das Verriegelungsglied 18a von der Schalthülse 17 für eine Bewegung nach innen sicher freigegeben. Durch die Federwirkung des Arms hintergreift das Verriegelungsglied 18a eine nach proximal gerichtete Kante des Autoinjektors oder rastet in eine axialfeste Ausnehmung des Autoinjektors ein und arretiert so die Sperrhülse 18 gegen eine distale Bewegung. Beim Entfernen des Autoinjektors von der Einstichstelle wird die Schalthülse 17 durch die Nadelschutzfeder 15 in distaler Richtung über das Verriegelungsglied 18a geschoben, worauf das Verriegelungsglied durch die Federwirkung des Arms in einer Verriegelungsposition eine nach proximal gerichtete Kante der Schalthülse 17 hintergreift und die Schalthülse sowie die Nadelschutzhülse gegen eine erneute Bewegung in proximale Richtung verriegelt oder blockiert.

Eine Kupplungshülse 23 mit zwei Haltenocken 23a ist über Kopplungselemente mit der Federspule 20b gekoppelt. Vor der Ausschüttung greifen die Haltenocken 23a in Ausnehmungen des axialfesten Mechanikhalters 13 ein und sind durch einen Innenumfang der Sperrhülse 18 an einer Bewegung nach aussen gehindert, wodurch sich die Kupplungshülse 23 auch axial nicht bewegen kann. Beim Auslösen der Ausschüttung wird die Sperrhülse 18 durch eine proximale Bewegung der Nadelschutzhülse 14 von der Position der Ausnehmungen wegbewegt, so dass sich die Haltenocken 23a radial lösen können und die Kupplungshülse 23 freigeben wird. Letztere bewegt sich in proximaler Richtung und gibt die Federspule 20b zur Rotation frei, wie ausführlich beschrieben in der Patentanmeldung PCTEP2021076923. Am proximalen Ende des Autoinjektors befindet sich die Anzeige mit einem rotierenden Anzeigeelement 25a mit einem Kontrastmuster in Form von parallelen Streifen, einem umlaufenden transparenten Fenster 25b, und einem proximalen, nichttransparenten Abschluss 25c.

Fig.3 zeigt zwei Ansichten des Anzeigeelements 25a mit einem Eingriffselement 25d zum Eingriff in ein nichtrotierendes Raster an der Innenseite des Fensters 25b oder des Abschlusses 25d. Eingriffselement 25d ist federnd gelagert und erzeugt bei Rotation des Anzeigeelements während der Ausschüttung eine der Rasterung entsprechende Anzahl von Klickgeräuschen. Das Anzeigeelement hat eine Form welche derjenigen des Fensters folgt beziehungsweise an diese angepasst ist, mit einem ersten, distalen Bereich und einem in proximaler Richtung daran anschliessenden zweiten Bereich. Der erste Bereich ist zylinderförmig, parallel zur Längsachse, während der zweite Bereich einen stetig abnehmenden Durchmesser aufweist.

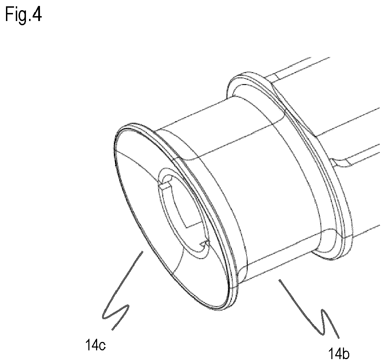

Fig.4 zeigt das distale Ende der Nadelschutzhülse mit dem hülsenartigen Abschnitt 14b und dem Flansch 14c. Deutlich zu sehen ist die Form des Flansches mit der konkav gewölbten Auflagefläche, deren äusserer, ovaler Rand gegenüber dem inneren Rand der Auflagefläche beim Übergang zum achsenparallelen Abschnitt nach distal vorsteht.

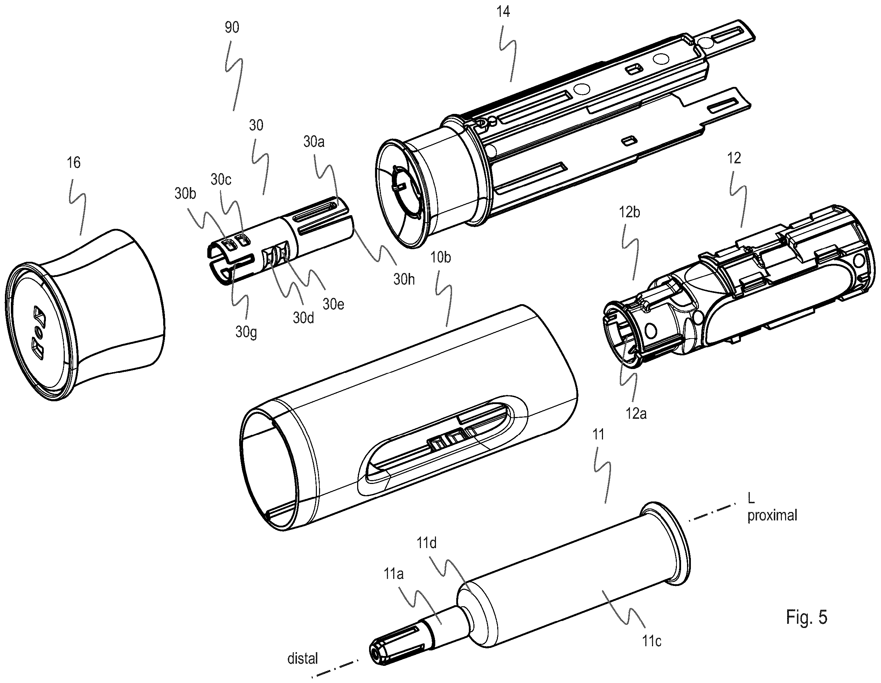

Fig.5 ist eine Explosionsdarstellung der Komponenten einer ersten Ausführungsform einer Produktbehälteraufnahmevorrichtung 90, umfassend eine Fertigspritze 11 mit einem Produktbehälter 11c welcher distal durch die Schulter 11d abschliesst und mit einer Nadelschutzkappe 11a welche die Injektionsnadel 11b umschliesst (nicht gezeigt), und umfassend eine Gerätekappe 16 welche einen Insert 30 aufnimmt. Am hülsenförmigen Insert 30 sind zwei elastische Schnapparme mit Schnapphaken 30a, je zwei erste und zweite Schnappnuten 30b, 30c je zwei Fenster als Verschnappungen 30d, 30e, zwei proximale Stirnseiten 30h sowie vier Nuten 30g geformt. In den Spritzenhalter 12 ist die Fertigspritze 11 durch die proximale Öffnung aufnehmbar und an der Innenseite der Führungshülse 12b sind zehn Längsrippen angebracht welche die Sperrfläche 12a ausbilden. Weiter sind die Nadelschutzhülse 14 sowie ein Gehäuseteil 10b gezeigt.

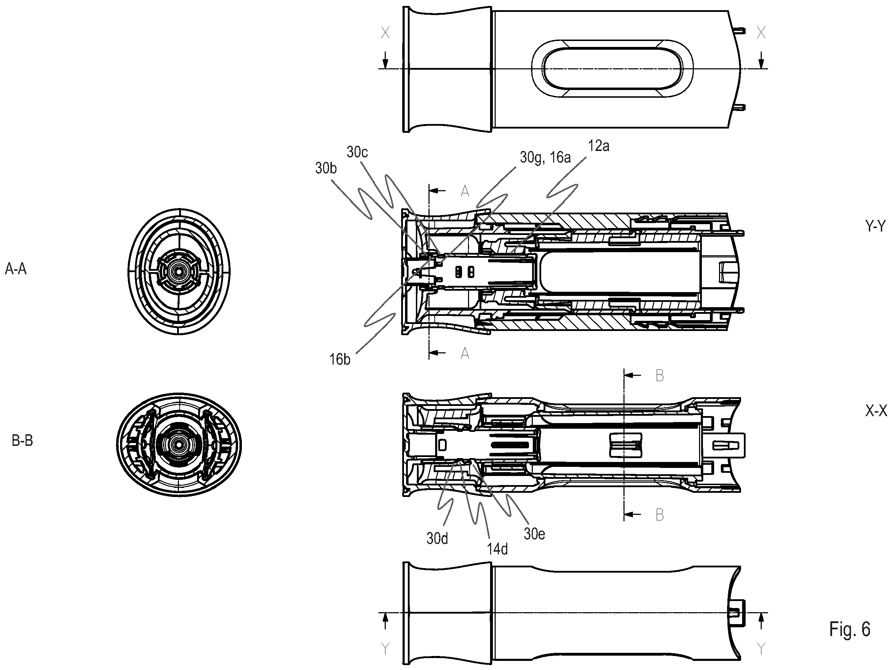

Fig.6 zeigt je zwei Längs- und Querschnitte der Vorrichtung aus Fig.5 im Zustand vor der Endmontage des Autoinjektors. Die Schnappnocken 16b im Inneren der Gerätekappe 16 greifen kraftschlüssig in die Schnappnuten 30b des Inserts 30. Weiter sind die vier Nuten 30g ersichtlich in denen eine Rippe 16a verschieblich aufgenommen ist. Die Sperrfläche 12a ist als Schnitt durch eine der zehn Rippen erkennbar. Die Nadelschutzhülse 14 greift mit ihren zwei Halteschnappern 14d in die zwei Öffnungen des Insert 30 und bildet so die erste Verschnappung 30d welche die Nadelschutzhülse und den Insert zueinander lösbar fixieren.

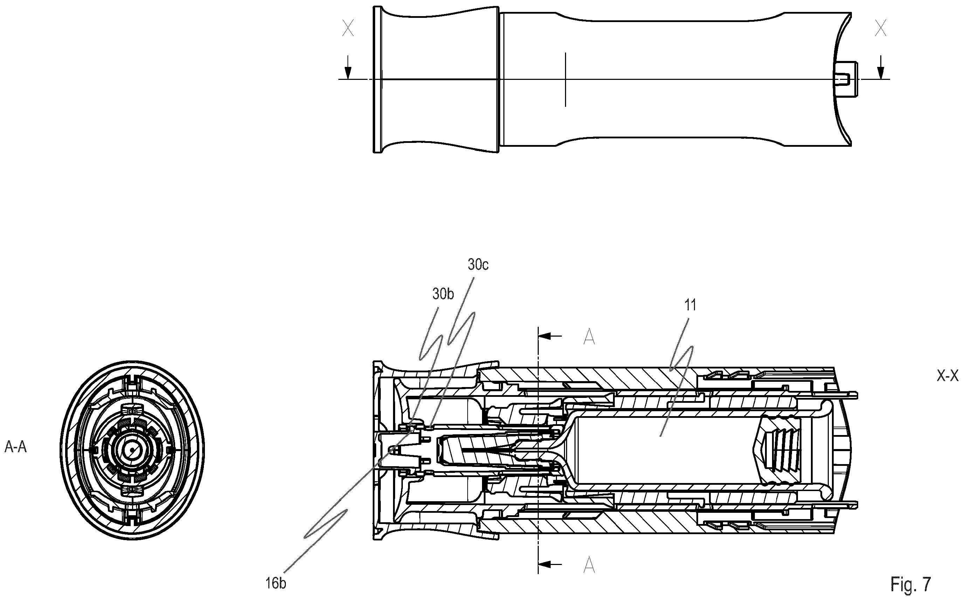

Fig.7 zeigt je ein Längs- und Querschnitt der Vorrichtung aus Fig.5 im Zustand des Fügens der Fertigspritze 11 unmittelbar vor der Sperrbewegung des Inserts 30. Die Schnappnocken 16b im Inneren der Gerätekappe 16 greifen kraftschlüssig in die Schnappnuten 30b des Inserts 30. Vor und zu Beginn der Fügebewegung sind die sind zwei elastischen Schnapparme mit Schnapphaken 30a nicht in Überdeckung mit der Sperrfläche 12a und so radial durch das äussere Profil der Nadelschutzkappe 11a auslenkbar bis die Schnapphaken 30a am proximalen Ende der Nadelschutzkappe 11a einfedern.

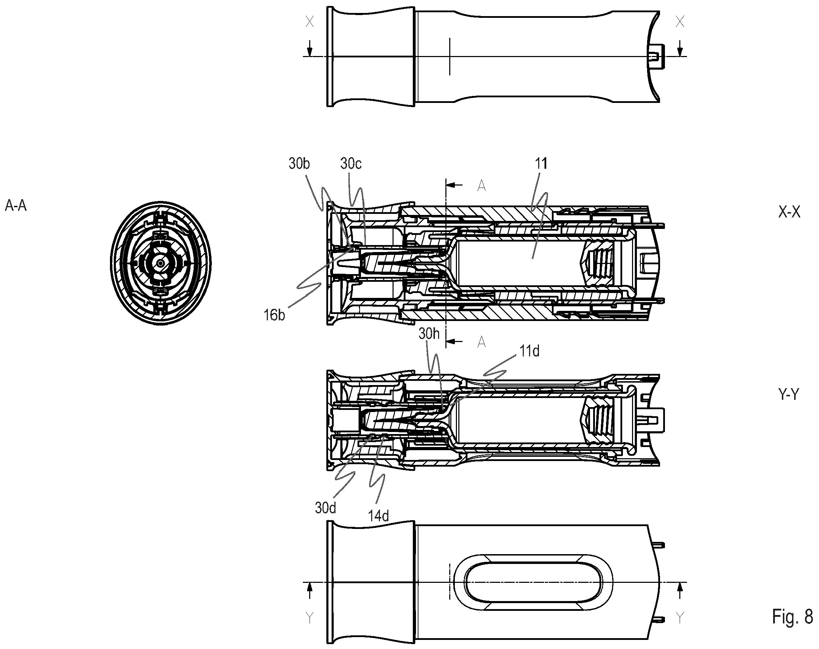

Fig.8 zeigt zwei Längs- und ein Querschnitt der Vorrichtung aus Fig.5 im Zustand nach der Sperrbewegung des Inserts 30. Die Sperrbewegung wird während dem Fügen von der Fertigspritze 11 bewirkt, indem diese mit der Schulter 11d den Insert 30 über dessen Stirnseiten 30h nach distal verschiebt wobei die zwei elastischen Schnapparme mit Schnapphaken 30a am Insert 30 in Überdeckung durch die Sperrfläche 12a gelangen und so gegen radiales Auslenken gesperrt werden. Die Schnappnocken 16b im Inneren der Gerätekappe 16 greifen nun nach der Sperrbewegung formschlüssig in die Schnappnuten 30c des Inserts 30 und die Nadelschutzhülse 14 greift nun mit ihren zwei Halteschnappern 14d in die zwei weiteren Öffnungen des Insert 30 und bildet so die zweite Verschnappung 30e welche die Nadelschutzhülse und den Insert zueinander lösbar fixieren.

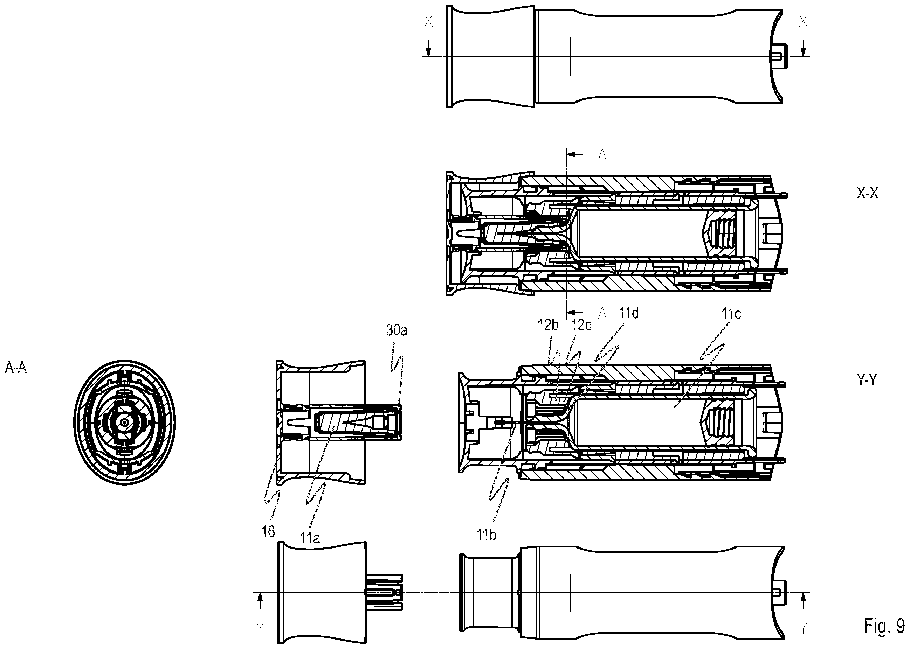

Fig.9 zeigt zwei Längs- und ein Querschnitt der Vorrichtung aus Fig.5 im Zustand nach dem Abziehen der Gerätekappe 16. Die Abzugskräfte werden von der Fertigspritze 11 über ihre Schulter 11d an den Anschlag 12c der Führungshülse 12b des Spritzenhalters 12 übertragen und von diesem auf das Gehäuse abgeleitet. Der Insert 30 bleibt beim Abziehen der Gerätekappe 16 aufgrund der formschlüssigen Verbindung der Schnappnocken 16b im Inneren der Gerätekappe 16 mit den Schnappnuten 30c des Inserts 30 fest mit der Gerätekappe verbunden und die zwei Schnapphaken 30a nehmen die Nadelschutzkappe 11a mit und trennen diese von der Fertigspritze 11. Somit wird die Injektionsnadel 11b freigelegt.

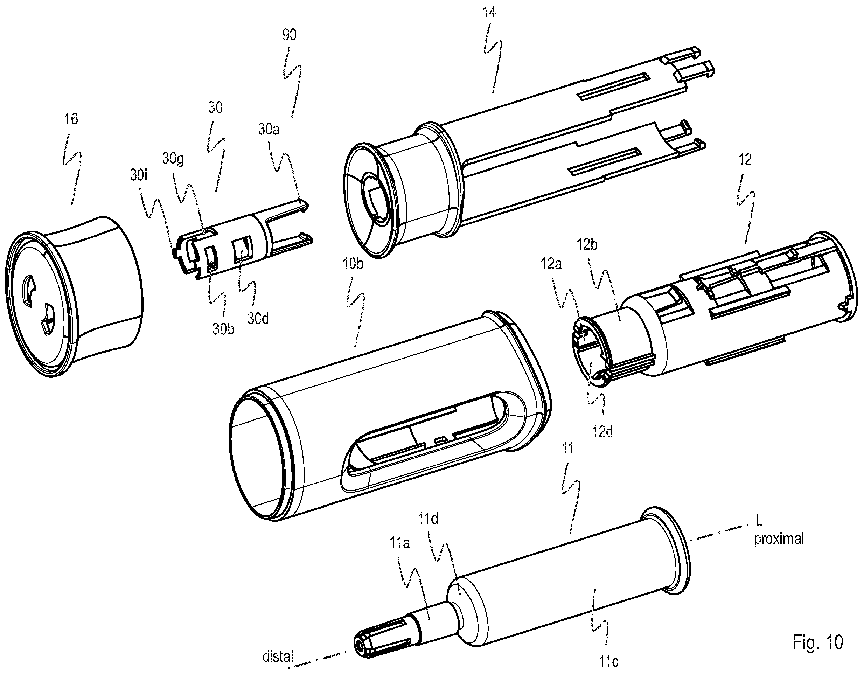

Fig.10 ist eine Explosionsdarstellung der Komponenten einer zweiten Ausführungsform einer Produktbehälteraufnahmevorrichtung 90. Die Fertigspritze 11 mit Produktbehälter 11c welcher distal durch die Schulter 11d abschliesst und mit einer Nadelschutzkappe 11a welche die Injektionsnadel 11b umschliesst (nicht gezeigt). Die Gerätekappe 16 welche den Insert 30 aufnimmt. Am hülsenförmigen Insert 30 sind zwei elastische Schnapparme mit Schnapphaken 30a, zwei Schnappnuten 30b, zwei Fenster als Verschnappungen 30d, zwei Nuten 30g sowie zwei Montagenocken 30i geformt. In den Spritzenhalter 12 ist die Fertigspritze 11 durch die proximale Öffnung aufnehmbar und an der Innenseite der Führungshülse 12b sind mehrere Längsrippen angebracht welche die Sperrfläche 12a ausbilden. Die Sperrfläche 12a ist durch zwei Ausnehmungen 12d unterbrochen. Weiter sind die Nadelschutzhülse 14 sowie ein Gehäuseteil 10b gezeigt.

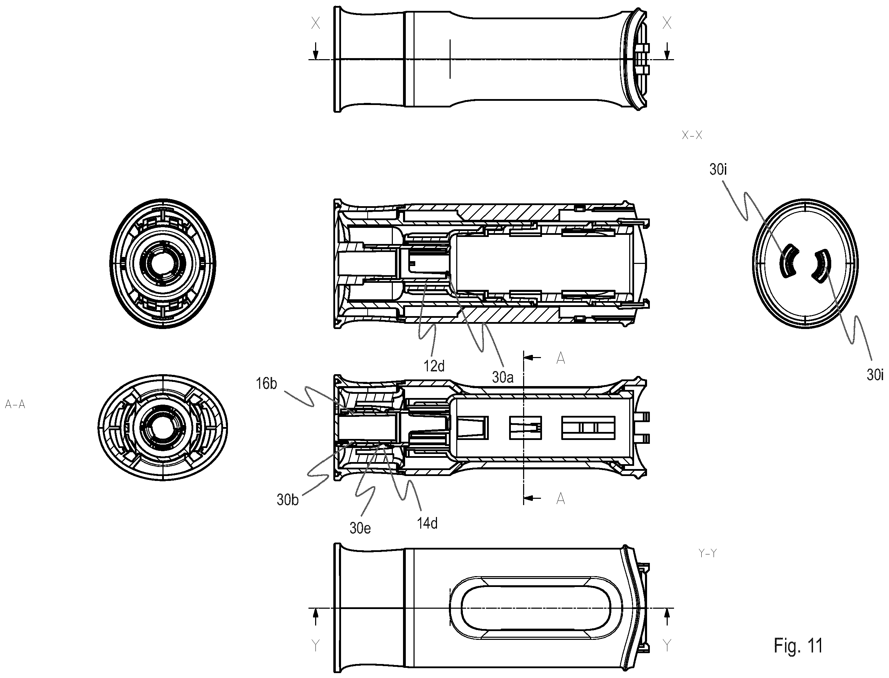

Fig.11 zeigt je zwei Längs- und Querschnitte der Vorrichtung aus Fig.10 im Zustand vor der Endmontage des Autoinjektors. Die zwei Schnappnocken 16b im Inneren der Gerätekappe 16 greifen formschlüssig in die Schnappnuten 30b des Inserts 30. Die Ausnehmung 12d der Sperrfläche 12a ist im Schnitt erkennbar und stellt die zwei elastischen Schnapparme mit Schnapphaken 30a für eine radiale Auslenkung frei. Die Nadelschutzhülse 14 greift mit ihren zwei Halteschnappern 14d in die zwei Öffnungen des Insert 30 und bildet so die erste Verschnappung 30d welche die Nadelschutzhülse und den Insert zueinander lösbar fixieren.

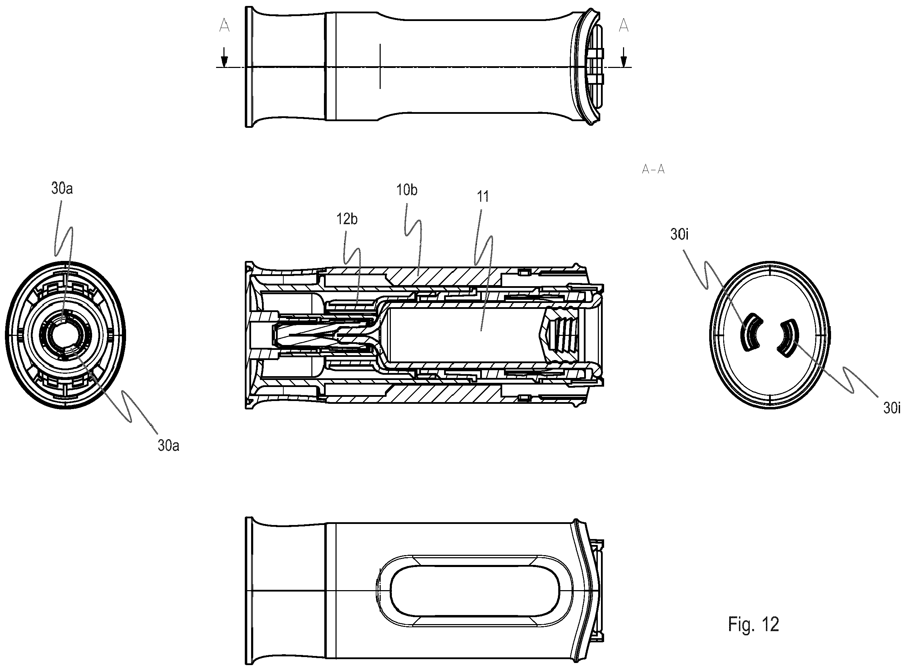

Fig.12 zeigt je ein Längs- und Querschnitt der Vorrichtung aus Fig.10 im Zustand des Fügens der Fertigspritze 11 unmittelbar vor der Sperrbewegung des Inserts 30. Die zwei Schnappnocken 16b im Inneren der Gerätekappe 16 greifen axial formschlüssig in die Schnappnuten 30b des Inserts 30 wobei der Insert 30 in der Gerätekappe drehbar bleibt. Die Montagenocken 30i sind über zwei Öffnungen in der Gerätekappe für ein Drehwerkzeug zugänglich und die die zwei elastische Schnapparme mit Schnapphaken 30a am Insert 30 sind im Bereich der Ausnehmung 12d und so radial durch das äussere Profil der Nadelschutzkappe 11a auslenkbar bis die Schnapphaken 30a am proximalen Ende der Nadelschutzkappe 11a einfedern.

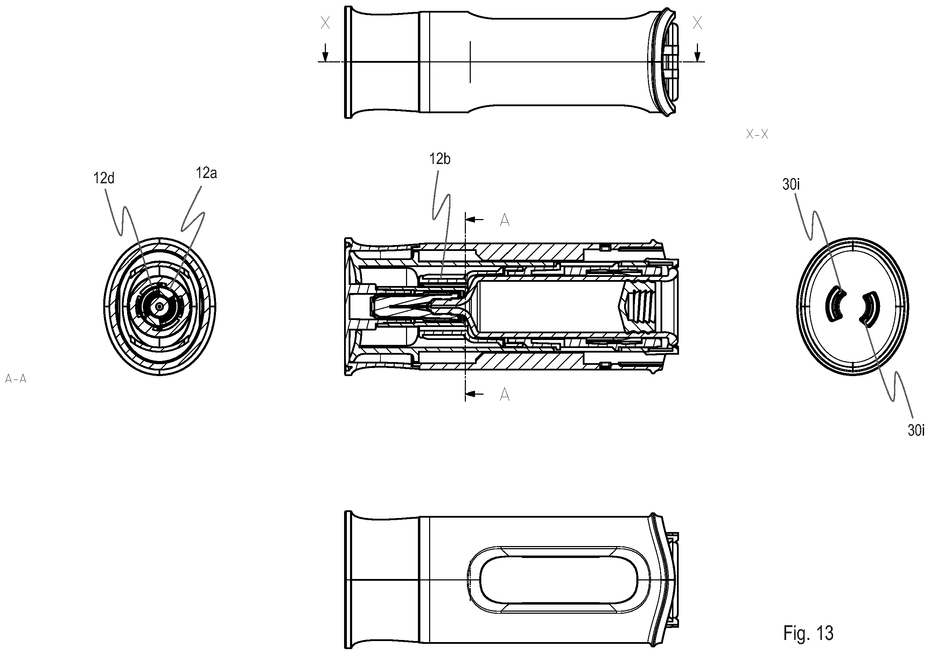

Fig.13 zeigt zwei Längs- und ein Querschnitt der Vorrichtung aus Fig.10 im Zustand nach der Sperrbewegung des Insert wobei der Insert 30 über die Montagenocken 30i gegenüber der Vorrichtung gedreht wurde, damit die zwei elastische Schnapparme mit Schnapphaken 30a am Insert 30 in Überdeckung durch die Sperrfläche 12a gelangen und so gegen radiales Auslenken gesperrt werden wobei diese neue Drehposition des Inserts 30 durch eine Schnappverbindung zwischen Gerätekappe 16 und der Nut 30g gesichert wird.

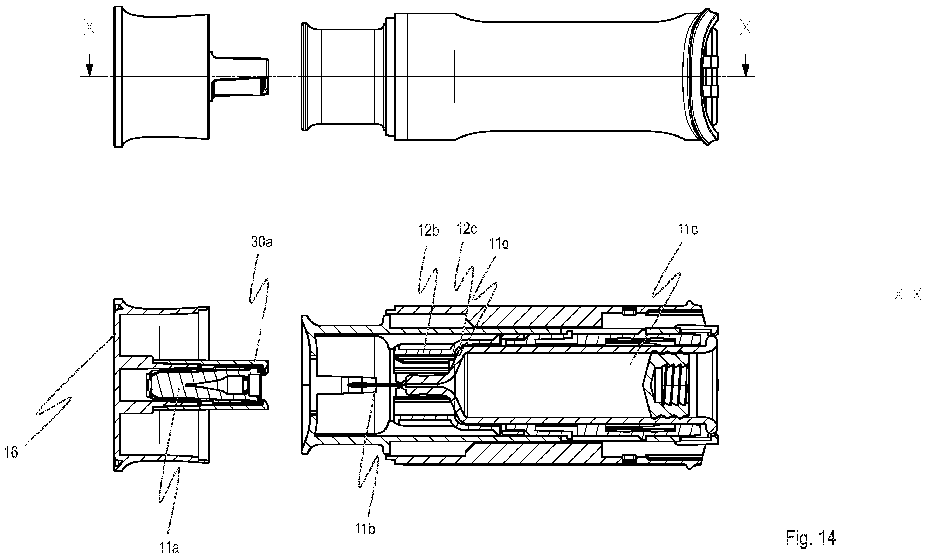

Fig.14 zeigt zwei Längs- und einen Querschnitt der Vorrichtung aus Fig.10 im Zustand nach dem Abziehen der Nadelschutzkappe. Die Abzugskräfte werden von der Fertigspritze 11 über ihre Schulter 11d an den Anschlag 12c der Führungshülse 12b des Spritzenhalters 12 übertragen und von diesem auf das Gehäuse abgeleitet. Der Insert 30 bleibt beim Abziehen der Gerätekappe 16 aufgrund der formschlüssigen Verbindung der zwei Schnappnocken 16b im Inneren der Gerätekappe 16 mit den Schnappnuten 30b des Inserts 30 fest mit der Gerätekappe verbunden und die zwei Schnapphaken 30a nehmen die Nadelschutzkappe 11a mit und trennen diese von der Fertigspritze 11. Somit wird die Injektionsnadel 11b freigelegt.

**BEZUGSZEICHENLISTE**

| | | | |
|---|---|---|---|
| 10a, 10b | Gehäuseteil | 16 | Gerätekappe |
| 10c | Ausnehmung | 17 | Schalthülse |
| 11 | Fertigspritze | 18 | Sperrhülse |
| 11a | Nadelschutzkappe | 18a | Verriegelungsglied |
| 11b | Injektionsnadel | 20a | Spiralfeder |
| 12 | Spritzenhalter | 20b | Federspule |
| 13 | Mechanikhalter | 21 | Antriebselement |
| 13a | Haltefederabschnitt | 22 | Vortriebselement |
| 13b | Federhülse | 23 | Kupplungshülse |
| 14 | Nadelschutzhülse | 23a | Haltenocken |
| 14a | Arm | 25a | Anzeigeelement |
| 14b | Abschnitt | 25b | Fenster |
| 14c | Flansch | 25c | Abschluss |
| 15 | Nadelschutzfeder | 25d | Eingriffselement |
| 30 | Insert | 14d | Halteschnapper |
| 30a | Schnapparm mit Schnapphaken | 16a | Rippe |
| 30b | Erste Schnappnut | 16b | Schnappnocke |
| 30c | Zweite Schnappnut | 16c | Rastnocke |
| 30d | Erste Verschnappung | | |
| 30e | Zweite Verschnappung | | |
| 30f | Führungsnut | | |
| 30g | Nut | | |
| 30h | Stirnseite | | |
| 30i | Montagenocken | L | Längsachse |
| 11c | Zylinderförmiger Produktbehälter | 1 | Autoinjektor |
| 11d | Schulter | | |
| 12a | Sperrfläche | | |
| 12b | Führungshülse | | |
| 12c | Anschlag | | |
| 12d | Ausnehmung | | |
| 90 | Produktbehälteraufnahmevorrichtung | | |

## Patentansprüche

1. Produktbehälteraufnahmevorrichtung (90) für einen Autoinjektor umfassend
- einen Spritzenhalter (12) ausgebildet zur Aufnahme einer vorgefüllten Fertigspritze (11) definierend eine Längsachse (L) welche sich von distal nach proximal erstreckt und aufweisend einen zylinderförmigen Produktbehälter (11c) welcher sich an seinem distalen Ende über eine Schulter (11d) verjüngt sowie eine Nadelschutzkappe (11a), wobei der Spritzenhalter (12) eine distal koaxial angeordnete Führungshülse (12b) aufweist, an deren Innenwand eine Sperrfläche (12a) ausgebildet ist,
- einen hülsenförmigen Insert (30) aufweisend einen radial bezüglich der Längsachse (L) auslenkbaren Schnapparm mit Schnapphaken (30a),
- eine Gerätekappe (16) in welcher der Insert (30) verbindbar aufgenommen ist, wobei der Insert (30) von einer ersten in eine zweite Rastposition relativ zur Gerätekappe (16) bewegbar ist und die Verbindung der Gerätekappe (16) mit dem Insert (30) in der zweiten Rastposition rastend und formschlüssig auf Zugkräfte entlang der Längsachse (L) ausgebildet ist,
**dadurch gekennzeichnet, dass** der Schnapparm mit Schnapphaken (30a) in der ersten Rastposition radial auslenkbar ist und in der zweiten Rastposition durch die Sperrfläche (12a) an einer radialen Auslenkung gehindert wird.

2. Produktbehälteraufnahmevorrichtung (90) nach dem vorhergehenden Anspruch, **dadurch gekennzeichnet, dass** der Schnapparm mit Schnapphaken (30a) in der ersten Rastposition des Inserts (30) durch die Nadelschutzkappe (11a) radial nach Aussen auslenkbar ist.

3. Produktbehälteraufnahmevorrichtung (90) nach einem der vorhergehenden Ansprüche, **dadurch gekennzeichnet, dass** der Schnapparm mit Schnapphaken (30a) dazu ausgebildet ist, in der zweiten Rastposition des Inserts (30) die Nadelschutzkappe (11a) in distale Richtung formschlüssig axial mitzunehmen.

4. Produktbehälteraufnahmevorrichtung (90) nach einem der vorhergehenden Ansprüche, **dadurch gekennzeichnet, dass** der Spritzenhalter (12) so ausgebildet ist, dass ein distaler Abschnitt der Fertigspritze (11) insbesondere die Nadelschutzkappe (11a) in der Führungshülse (12b) axial bewegbar ist und diese Bewegbarkeit der Fertigspritze (11) in distaler Richtung formschlüssig begrenzt ist.

5. Produktbehälteraufnahmevorrichtung (90) nach dem vorhergehenden Anspruch, **dadurch gekennzeichnet, dass** die Führungshülse (12b) mit ihrem proximalen Ende einen Anschlag (12c) bildet für den zylinderförmigen Produktbehälter (11c) insbesondere für dessen Schulter (11d).

6. Produktbehälteraufnahmevorrichtung (90) nach einem der vorhergehenden Ansprüche, weiter umfassend eine Nadelschutzhülse (14) mit einem Halteschnapper (14d) **dadurch gekennzeichnet, dass** in der ersten Rastposition der Halteschnapper (14d) den Insert (30) durch Eingriff in eine erste Verschnappung (30d) kraftschlüssig relativ zur Nadelschutzhülse (14) verbindet und/oder in der zweiten Rastposition der Halteschnapper (14d) den Insert (30) durch Eingriff in eine zweite Verschnappung (30e) oder durch Bewegung in der ersten Verschnappung (30d) kraftschlüssig relativ zur Nadelschutzhülse (14) verbindet.

7. Produktbehälteraufnahmevorrichtung (90) nach dem vorhergehenden Anspruch, **dadurch gekennzeichnet, dass** die Gerätekappe (16) von der Produktbehälteraufnahmevorrichtung (90) durch eine Abzugsbewegung trennbar ist, wobei der Insert (30) mitgenommen wird und die kraftschlüssige Verbindung (14d, 30e) zwischen Nadelschutzhülse (14) und Insert (30) gelöst wird.

8. Produktbehälteraufnahmevorrichtung (90) nach einem der vorhergehenden Ansprüche, weiter umfassend ein Gehäuse oder Gehäuseteil (10a, 10b) **dadurch gekennzeichnet, dass** der Spritzenhalter (12) unbeweglich zumindest auf Zug in distaler Richtung oder Druck aus proximaler Richtung in diesem aufgenommen ist oder einstückig mit diesem gebildet ist.

9. Produktbehälteraufnahmevorrichtung (90) nach einem der vorhergehenden Ansprüche, **dadurch gekennzeichnet, dass** der Insert (30) von der ersten in die zweite Rastposition relativ zur Gerätekappe (16) axial von proximal nach distal bewegbar ist und eine Schnappnocke (16b) der Gerätekappe (16) von einer ersten Schnappnut (30b) des Inserts (30) in eine zweite Schnappnut (30c) wechselt.

10. Produktbehälteraufnahmevorrichtung (90) nach dem vorhergehenden Anspruch, wobei der Insert (30) über eine proximal vorgesehene Stirnseite (30h) durch die Schulter (11d) axial in distale Richtung drängbar ist.

11. Produktbehälteraufnahmevorrichtung (90) nach den zwei vorhergehenden Ansprüchen, **dadurch gekennzeichnet, dass** die Sperrfläche (12a) die an der Innenwand der Führungshülse (12b) ausgebildet ist auf einen axialen Abschnitt begrenzt ist.

12. Produktbehälteraufnahmevorrichtung (90) nach einem der vorhergehenden Ansprüche 1 bis 8, **dadurch gekennzeichnet, dass** der Insert (30) von der ersten in die zweite Rastposition relativ zur Gerätekappe (16) drehend um die Längsachse (L) bewegbar ist und durch eine Schnappnocke (16b) in einer Schnappnut (30b) axial fest geführt ist und/oder durch die Nut (30g) in der zweiten Rastposition drehfest sicherbar ist.

13. Produktbehälteraufnahmevorrichtung (90) nach dem vorhergehenden Anspruch, **dadurch gekennzeichnet, dass** der Insert (30) über einen oder mehrere distal vorgesehene Montagenocken (30i) drehend um die Längsachse bewegbar ist.

14. Produktbehälteraufnahmevorrichtung (90) nach den zwei vorhergehenden Ansprüchen, **dadurch gekennzeichnet, dass** die an der Innenwand der Führungshülse (12b) ausgebildete Sperrfläche (12a) sektorweise eine oder mehrere Ausnehmungen (12d) aufweist.

15. Autoinjektor (1) für die Verabreichung eines flüssigen Produkts umfassend
- eine Produktbehälteraufnahmevorrichtung (90) nach einem der Ansprüche 1 - 14
- ein Vortriebsglied (22) und ein Antriebselement (21) zur Bewegung des Vortriebsglieds (22) in Längsrichtung und zur automatischen Ausschüttung eines in dem Produktbehälter (11c) der vorgefüllten Fertigspritze (11) enthaltenen flüssigen Produkts durch eine Injektionsnadel (11b) wobei die Nadelschutzhülse (14), welche im Auslieferzustand des Autoinjektors (1) distal über das Gehäuse (10a,10b) vorsteht, bei Anpressen des Autoinjektors (1) an eine Injektionsstelle eine Betätigungsbewegung in proximale Richtung ausführt und bei Entfernen des Autoinjektors (1) von der Injektionsstelle eine Nadelschutzbewegung in distaler Richtung ausführt, um mit einem hülsenförmigen Abschnitt (14b) die Injektionsnadel (11b) seitlich zu umgeben.
